# EUROPEAN PATENT APPLICATION

(11) **EP 1 941 883 A1**
(43) Date of publication of application: **09.07.2008**
(21) Application number: 07000267.0
(22) Date of filing: 08.01.2007
(51) Int. Cl.: A61K 31/4965, A61P 11/00

(54) **Use of sodium blockers for an early therapy of obstructive lung diseases**

(71) Applicant: Universität Heidelberg, 69117 Heidelberg (DE)
(72) Inventor: Mall, Marcus, Dr., 69117 Heidelberg (DE)
(74) Representative: Müller-Boré & Partner Patentanwälte

(57) **Abstract**

The present invention relates to a blocker of sodium channels in cell membranes, particularly in membranes of epithelial cells of organs belonging to the respiratory tract to be used as the pharmaceutically active ingredient in a medicament for treating an obstructive lung disease in a patient.

## Description

The present invention relates to a blocker of sodium channels in cell membranes, particularly in membranes of epithelial cells of organs belonging to the respiratory tract to be used as the pharmaceutically active ingredient in a medicament for treating an obstructive lung disease in a patient.

Obstructive lung diseases like cystic fibrosis (CF), neonatal chronic lung disease (CLD), also known as bronchopulmonary dysplasia (BPD), asthma bronchiale and chronic bronchitis (also known as chronic obstructive pulmonar disease; COPD) belong to the most common chronic diseases in Western Europe and North America. While CF is the most common fatal hereditary disease in the white population, CLD is a frequent health problem of premature infants. Asthma bronchiale is one of the most common chronic diseases of children and asults. Cigarette smoke induced COPD is currently the fourth leading cause of death worlwide. All chronic obstructive lung diseases are accompanied by various degrees of mucus obstructions, goblet cell metaplasia and chronic inflammation of the respiratory tract and the formation of emphysemas, i.e. disturbance in development or a destruction of alveoli, resulting in a respiratory insufficiency. To this date only limited therapies, which are primarily oriented on the symptoms, like e.g. administration of β-mimetics, corticosteroids, anticholinergics, antibiotics, and mucolytics to a patient and physiotherapy are available for the therapy of these diseases. Therefore, a new effective therapy of obstructive lung diseases is of high clinical and socioeconomic interest.

In the respiratory tract of CF patients there is a defect in the cAMP-dependent secretion of chloride and an enhancement in the resorption of sodium (Knowles M.R. et al., Science 1983;221:1067-1070). These characteristic defects of the epithelial transport of ions leads primarily to a dehydration (depletion of volume) of the surface of the respiratory tract and therefore to a defect of the mucociliar clearance and the pulmonal defense. In the case of asthma, CLD, COPD, and most of the other obstructive lung diseases, there is primarily an inflammation of the respiratory tract with mucus hypersecretion which results in a secondary dehydration of the surface of the respiratory tract and therefore also results in a defect of the mucociliary clearance.

The importance of the absorption of sodium in the *in vivo* pathogenesis of chronic obstructive lung diseases has also been shown in transgenic mouse models (Mall M et al., Nat Med 2004;10:487-493). It should be noted that overexpression of the β-subunit of the epithelial sodium channel (ENaC, also known as SCNN1) in the respiratory tract of mice also results in spontaneous lung diseases having a great similarity to CF as well as to other chronic obstructive lung diseases of human beings (CF, CLD, asthma bronchiale, chronic bronchitis, COPD). This means that the respiratory tract of β-ENaC overexpressing mice is dehydrated on the surface which leads to a defect of the mucociliary clearance, mucus obstruction, goblet cell metaplasia, chronic inflammation, and the formation of emphysema.

Further, the above observation, i.e. that a dehydration of the surface of the respiratory tract of CF patients leads to a chronic obstructive lung disease, forms the rationale for a therapy with specific inhibitors of the epithelial sodium channels. This strategy should inhibit the resorption of liquids by the surface of the respiratory tract so that the hydration of the surface film and the mucociliar clearance is improved and therefore antagonizes the mucus obstruction. Recent tests for the therapeutic effectiveness of an aerosol of the classic sodium channel blocker amiloride for the treatment of the lung disease of CF-patients, however, did not show any therapeutic effect (Graham A. et al., Eur Respir J 1993;6:1243-1248; Bowler IM et al., Arch Dis Child 1995;73:427-430; Pons G. et al., Pediatr Pulmonol 2000;30:25-31).

So far the aerosol therapy with the sodium channel blocker amiloride has been exclusively carried out for CF patients having an advanced lung disease, wherein the minimum age of the patients in said studies was five years. In this group of patients the treatment with amiloride had no therapeutic success.

Thus, the problem underlying the present invention is to provide a new method for a successful *in vivo* therapy of obstructive lung diseases using sodium channel blockers.

The solution to the above technical problem is achieved by the embodiments characterized in the claims.

In particular, the present invention relates to the use of at least one sodium channel blocker as a pharmaceutically active ingredient of a medicament for treating an obstructive lung disease in a susceptible patient prior to having substantial mucus obstruction or secondary, disease related changes of the lung. Therefore, in one embodiment the present invention relates to the use of at least one sodium channel blocker as a pharmaceutically active ingredient of a medicament for an early therapy of an obstructive lung disease in a patient, wherein the early therapy is carried out in an early phase of the disease characterized by the lack of any substantial mucus obstruction or secondary, disease related changes of the lung.

The sodium channel to be blocked may be any sodium channel in cell membranes, particularly in membranes of epithelial cells of organs belonging to the respiratory tract. The organ of the respiratory tract may be for example the trachea or the lung including bronchi, bronchioles, and alveoli. In a preferred embodiment of the present invention, the sodium channels to be blocked are situated in membranes of epithelial cells of the lung.

The term "medicament" as used herein relates to any pharmaceutical composition comprising at least one sodium channel blocker in a pharmaceutically effective amount.

According to the present invention, the medicament may be administered by any administration route known in the art being suitable for delivering a medicament to the epithelium of an organ belonging to the respiratory tract. The route of administration does not exhibit particular limitations and includes for example inhalation, e.g. intrapulmonal, or nasal administration, systemic administration, e.g. by oral or intravenous route, and topic application, e.g. as an ointment. The medicament may be administered in any form known in the art, e.g. as a liquid, a powder, an aerosol, a capsule, or a tablet.

In a preferred embodiment of the present invention, the medicament is administered by an intrapulmonal application as an aerosol. The medicament according to the present invention may be for example inhaled, e.g. by using special devices or nebulizers, which administer the medicament in a fine spray that the patient breathes in. Further, suitable inhalers, which may be used for administering the medicament, like e.g. a metered-dose inhaler (MDI), which allows precise doses to be delivered directly to the lungs, are known to those skilled in the art. Such inhalers may use for example ozone-depleting chlorofluorocarbons or hydrofluoroalkane as propellants, but alternative delivery methods and propellants useful for delivering the medicament, like e.g. dry powder inhalers (DPIs), may also be used.

The term "early therapy" as used herein relates to a therapy that is initiated in a susceptible individual prior to the development of a lung disease (i.e. preventive) or in an early stage of an obstructive lung disease. This may be a preventive treatment that is initiated in a susceptible individual before the onset of a lung disease or a therapy for the treatment of an early stage of an obstructive lung disease, characterized e.g. by no progressed lung disease like mucus obstruction and no secondary changes of the lung, like e.g. airway remodelling, goblet cell metaplasia, chronic inflammation of the respiratory tract or emphysema, which may be evidenced e.g. by standard diagnostic tests including pulmonary function testing, pulmonary imaging, bronchoscopy with bronchoalveolar lavage.

Patients who are known to be susceptible to develop a chronic obstructive lung disease and will therefore benefit from a preventive or early therapy can be identified depending on the disease etiology as follows:

CF is an inherited multiorgan disease caused by mutations in the CRTR gene. Lungs are normal at birth and the disease typically presents with gastrointestinal symptoms like meconium ileus, malabsorption and maldigestion due to pancreatic insufficiency, and failure to growth, i.e. the clinical diagnosis can often be established and confirmed by standard laboratory tests like sweat test or genetic testing in infancy prior to the onset of lung disease. Some patients are already identified before birth by prenatal screening for CFTR mutations. Further, neonatal CF screening programmes are currently being established in many countries that will allow to identify CF patients in the first weeks of life. Taken together, the majority of CF patients in the Western world are identified before the onset of chronic lung disease.

Neonatal CLD is caused by premature birth, i.e. patients at risk are readily identified and treatment can be commenced right after birth, i.e. before lung disease has developed.

Asthma is an episodic, recurrent disease characterized by reversible airway obstruction caused by various triggers including viral infections, allergens, physical exercise, or cold air. Typically, acute and recurrent episodes with reversible airflow obstruction due to mucus obstruction, goblet cell metaplasia, airway inflammation and related smooth muscle contraction alternate with symptom free episodes with no mucus obstruction, goblet cell metaplasia or inflammation in the absence of the trigger. Accordingly, "preventive or early therapy" could be commenced in a symptom free interval and preferably prevent or ameliorate the next asthma attack.

COPD typically starts in adulthood and is caused by chronic inhalation of cigarette smoke or other noxious particulates and/or toxicants. Since the establishment of chronic obstructive lung disease including mucus obstruction, inflammation, goblet cell metaplasia and emphysema takes several years, individuals at risk can be readily identified prior to the onset of lung disease, and the treatment commenced as a "preventive or early therapy" in individuals who smoke cigarettes or are exposed to occupational particulates and/or toxicants.

As used herein, a sodium channel blocker may be any molecule that is able to substantially decrease the ability of a sodium channel to transport sodium ions from the extracellular side of a cell membrane into the intracellular side of a cell membrane. In a preferred embodiment of the present invention, the sodium channel blocker is selected from the group consisting of amiloride, amiloride analogs, P2Y2-receptor agonists such as nucleotides, like e.g. ATP or UTP, or long-acting synthetic compounds like nucleotide analogs (e.g. Denufosol), and protease inhibitors, including e.g. aprotinin or BAY 39-9437 (a recombinant Kunitz-type serine protease inhibitor) or derivatives thereof. In a particularly preferred embodiment of the present invention, the sodium channel blocker is amiloride (3,5-Diamino-N-(aminoiminomethyl)-6-chloro-pyrazinecarboxamide) or a derivative thereof. The term "derivative thereof' as used herein includes any derivative of a sodium channel blocker having substantially the same functional, such as biological and/or pharmacological, properties as the non-derivatized sodium channel blocker, i.e. to effectively block sodium channels.

According to the present invention, the sodium channel blocker is used in a pharmaceutically effective amount. In a preferred embodiment of the present invention the sodium channel blocker is used in a amount ranging from about 0.1 mg/kg body weight to about 10 mg/kg body weight. In a more preferred embodiment of the present invention, amiloride is used in a amount ranging from about 0.3 mg/kg body weight to about 1 mg/kg body weight. In another more preferred embodiment of the present invention a P2Y2-receptor agonist is used in a amount ranging from about 1 mg/kg body weight to about 2 mg/kg body weight. In a further more preferred embodiment of the present invention a protease inhibitor is used in a amount ranging from about 0.3 mg/kg body weight to about 1 mg/kg body weight.

The term "obstructive lung disease" as used herein relates to a disease characterized by airflow limitation in the lung that develops over time. The obstructive lung disease according to the present invention may be associated with breathing-related symptoms, like e.g. cough, spitting or coughing mucus (expectoration), breathlessness upon exertion, progressive reduction in the ability to exhale, progressive shortness of breath, frequently accompanied by a phlegm-producing cough, with episodes of wheezing, irritation of the nose and throat, chest tightness or pain or a nonproductive cough. The above symptoms may vary, however, others may be present.

Examples of the obstructive lung disease according to the present invention are acute bronchitis which is usually caused by a virus and in most cases is self-limiting but can later develop either chronic bronchitis or asthma, and asthma which is characterized by attacks of coughing, wheezing, and shortness of breath (dyspnea). In a preferred embodiment of the present invention the obstructive lung disease is a chronic obstructive lung disease. An example of such a chronic obstructive lung disease is chronic bronchitis (COPD) being characterized by chronic cough and sputum production, intermittent wheezing with variable degrees of shortness of breath on exertion. Other examples of chronic obstructive lung diseases are cystic fibrosis (CF) characterized by an increased transport of sodium across the respiratory tract lining which results in the dehydration of the liquid that lines the respiratory tract surface and neonatal chronic lung disease (CLD).

In another preferred embodiment of the present invention the obstructive lung disease is selected from the group consisting of cystic fibrosis (CF), neonatal chronic lung disease (CLD), asthma bronchiale, and chronic bronchitis. In a more preferred embodiment of the present invention the obstructive lung disease is CF.

Further, the term "treatment" as used herein relates to the prevention and/or eradication or amelioration of disease related symptoms and/or disease related disorders. Obstructive lung diseases are often accompanied by pulmonal mortality, chronic inflammation of the respiratory tract, like e.g. pulmonal inflammation, mucus obstruction, resulting from secreted mucus, a viscous fluid composed primarily of highly glycosylated proteins called mucius suspended in a solution of electrolytes. Other disorders associated with obstructive lung diseases are goblet cell hyperplasia, goblet cell metaplasia being an important morphological feature in the respiratory tract of patients with chronic respiratory tract diseases, and emphysema which is a progressive destructive lung disease in which the walls between the alveoli in the lungs are damaged. Therefore, a preferred embodiment of the present invention is a use of at least one sodium channel blocker in the manufacture of a medicament for an early therapy of an obstructive lung disease as described above, wherein at least one disorder selected from the group consisting of pulmonal mortality, pulmonal inflammation, mucus obstruction, goblet cell metaplasia, and emphysema is reduced in the patient, e.g. when compared to patients not being treated with a sodium channel blocker according to the present invention.

The reduction of the above symptoms and disorders as well as the success of an early therapy of an obstructive lung disease in a patient by use of a sodium channel blocker as described above can be monitored using methods known in the art. Examples of such methods for determining the presence and the course of the response to treatment of obstructive lung diseases are pulmonary function tests, like e.g. spirometry employing a spirometer, an instrument that measures the air taken into and exhaled from the lungs, or the testing of arterial blood gas by determining the amount of oxygen and carbon dioxide in the blood; wherein low oxygen (hypoxia) and high carbon dioxide (hypercapnia) levels are often indicative of chronic bronchitis and emphysema. Another example is the lung carbon monoxide diffusing capacity (DLCO) test which determines how effectively gases are exchanged between the blood and the respiratory tract in the lungs. Further, imaging tests, like e.g. chest x-rays or computed tomography (CT) scans, and tests for the protective enzyme, alpha 1-antiprotease (ATT or antitrypsin) which is often deficient in patients having an obstructive lung disease, and bronchoalveolar lavage for determination of inflammatory cells and pro-inflammatory cytokines in the lung may be employed.

The early therapy of an obstructive lung disease in a patient by use of a sodium channel blocker as described above can also be combined with any therapy known in the art for the therapy of an obstructive lung disease. Accordingly, the present invention also relates to the use of at least one sodium channel blocker in the manufacture of a medicament which may also contain further active agents like e.g. anticholinergic agents which relax the bronchial muscles and act as a bronchodilator when inhaled, beta2 agonists being bronchodilators, theophylline, which acts by opening the respiratory tract, improving exchange of gases, reducing shortness of breath, improving mucus clearance, and stimulating the process of breathing, corticosteroids being anti-inflammatory drugs, and osmotically active agents including hypertonic saline or mannitol that improve airway surface hydration by their osmotic action.

The term "patient" as used herein does not underly any specific limitation and includes mammals. In a preferred embodiment of the present invention, the patient is a human.

The present invention further relates to a method of treating a patient having an obstructive lung disease as defined above with at least one sodium channel blocker as defined above, wherein the sodium channel blocker is administered in an early therapy as defined above.

The figures show:
Figure 1 shows that early amiloride treatment (started on the first day of life and continued for 14 days) significantly improved survival of βENaC-transgenic mice compared to vehicle treated βENaC-transgenic littermates. H₂O was used as vehicle in all experiments. Wt, wild-type; tg, βENaC-transgenic. n = 35-48 mice per group. **P* = 0.004.
Figure 2 shows that late amiloride treatment (started on postnatal day 5 and continued for 14 days) had no effect on survival of βENaC-transgenic mice compared to vehicle treated βENaC-transgenic littermates. Wt, wild-type; tg, βENaC-transgenic. n = 17-34 mice per group.
Figure 3 shows that early amiloride treatment (started on first day of life and continued for 14 days) significantly reduced bronchoalveolar lavage (BAL) eosinophil cell counts in βENaC-transgenic mice compared to vehicle treated βENaC transgenic littermates. Means ± SEM, n = 16-34 mice per group. **P* = 0.002.
Figure 4 shows that late amiloride treatment (started on postnatal day 5 and continued for 14 days) had no effect on BAL inflammatory cell counts in βENaC transgenic mice compared to vehicle treated βENaC transgenic littermates. Means ± SEM, n = 13-34 mice per group
Figure 5 shows that BAL macrophages are activated ('foam cells') in vehicle treated βENaC transgenic mice compared to wild-type littermates. Early amiloride treatment (started on first day of life and continued for 14 days) reduced number of BAL foam cells and average macrophage diameters in βENaC transgenic mice. Giemsa staining. Representative for n = 16-34 mice per group. Scale bars = 20 µm.
Figure 6 shows that early amiloride treatment (started on first day of life and continued for 14 days) reduced severity of airway mucus plugging in βENaC-transgenic mice (right panel) compared to vehicle (H₂O) treated βENaC-transgenic littermates (middle panel). AB-PAS staining. Scale bars = 500 µm (wt), and 200 µm (tg) respectively. Representative for n = 16-34 mice per group.
Figure 7 shows that early amiloride treatment (started on first day of life and continued for 14 days) reduced severity of goblet cell metaplasia in βENaC transgenic mice compared to vehicle treated βENaC transgenic littermates. Means ± SEM, n = 14-33 mice per group. **P* < 0.001.

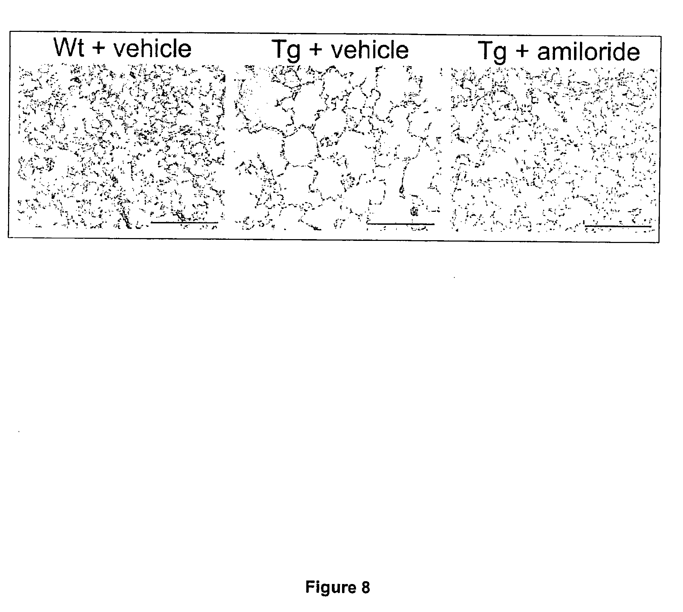

Figure 8 shows that early amiloride treatment (started on first day of life and continued for 14 days) reduced severity of emphysema in βENaC transgenic mice compared to vehicle treated βENaC transgenic littermates. H&E staining. Representative for n = 8-21 mice per group. Scale bars = 200 µm.

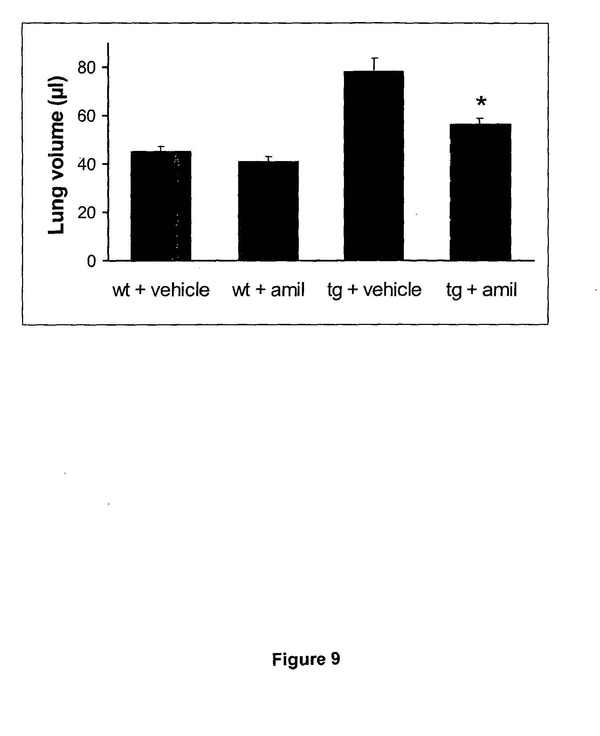

Figure 9 shows that early amiloride treatment (started on first day of life and continued for 14 days) reduced increased lung volume in βENaC transgenic mice compared to vehicle treated βENaC transgenic littermates. Means ± SEM, n = 8-21 mice per group. **P* < 0.001.

The present invention advantageously provides a therapy for the successful treatment of obstructive lung diseases by applying a specific sodium channel blocker like amiloride or a derivative thereof in a living organism as an early therapy. It has surprisingly been found that by the intrapulmonary application of a sodium channel blocker in an early stage of a diagnosed disease, the obstructive lung disease can be cured and disorders associated with said disease like e.g. pulmonal mortality, pulmonal inflammation, mucus obstruction, goblet cell metaplasia, and emphysema can be reduced. These superior results are achieved by an early therapy of the patients, i.e. at a stage of the disease when mucus obstruction has not been developed and further secondary changes of the lung, like e.g. goblet cell metaplasia, chronic inflammation of the respiratory tract or emphysema, are not apparent.

The present invention will now be further illustrated in the following examples without being limited thereto.

### Examples

The β-ENaC transgenic mouse has been used as an animal model for chronic obstructive lung diseases of humans to test, whether chronic obstructive lung disease can be treated successfully in a living organism by an early therapy with a sodium channel blocker, i.e. by starting treatment before the development of mucus obstruction and secondary changes of the lung occur. Similar to humans with chronic obstructive lung diseases including CF, CLD, asthma and COPD, the lungs of β-ENaC transgenic mice are normal at birth. Subsequently, β-ENaC transgenic mice develop a spontaneous lung disease that has great similarities to said chronic obstructive lung diseases in humans. At 5 days of age, β-ENaC transgenic mice have already developed significant mucus obstruction and airway inflammation that causes death due to respiratory failure in ~50% of β-ENaC transgenic mice in the first 2 weeks of live. For this reason, we treated β-ENaC transgenic mice either from the first day of their lives, i.e. from a date, wherein there were no changes of the lung, or from their fifth day of their lives, i.e. from a date at which mucus obstructions and inflammation of the respiratory tract already existed, with an intrapulmonal application of amiloride.

Intrapulmonal application of amiloride in neonatal mice was achieved by intranasal (i.n.) application of amiloride at a concentration of 3 g/l in a volume of 1 ml/kg body weight, equivalent to a dose of 3 mg/kg body weight. Water was used as vehicle and β-ENaC transgenic mice or wild-type littermate controls were treated three times per day with amiloride or vehicle alone for a period of 2 weeks. To prevent systemic side effects, i.e. possible dehydration due to inhibition of sodium channels in the kidney, in case part of the intranasally applied amiloride was swallowed and absorbed systemically, amiloride-treated mice received concomitant subcutaneous (s.c.) injections with isotonic sodium chloride solution (NaCl 0.9%).

Animals were monitored daily, and deceased mice were genotyped and mortality curves constructed for all treatment groups. At the end of the 2 week treatment cycle, surviving mice were euthanized, and lungs evaluated for several independent clinically relevant outcome measures, including bronchoalveolar lavage to determine therapeutic effects on pulmonary inflammatory cell counts; histopathology, morphometry and lung volume measurements determine effects on mucus obstruction, goblet cell metaplasia and emphysema.

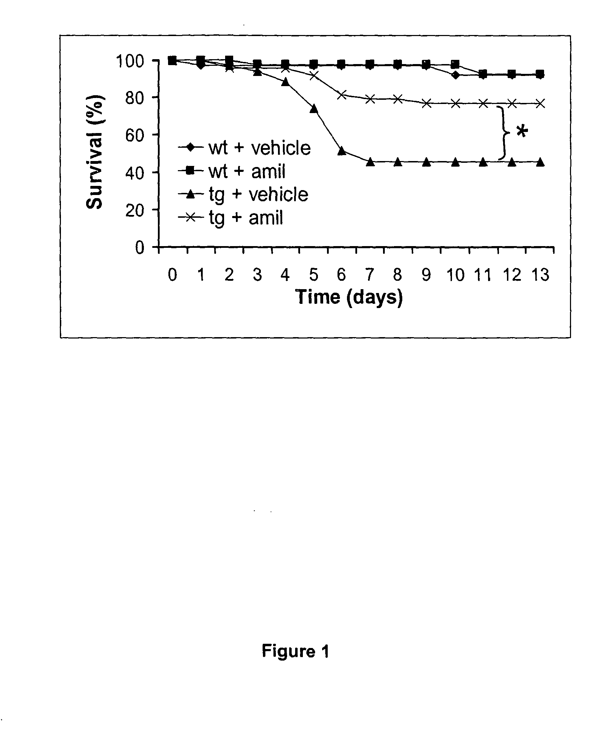

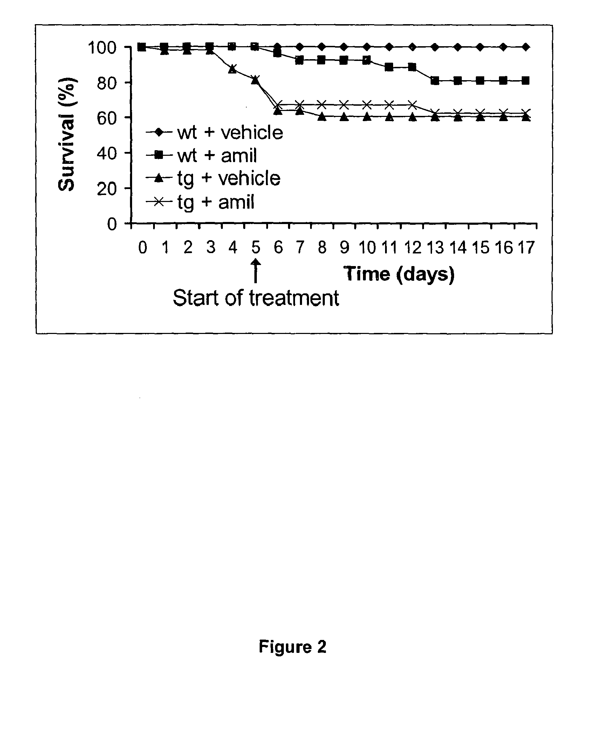

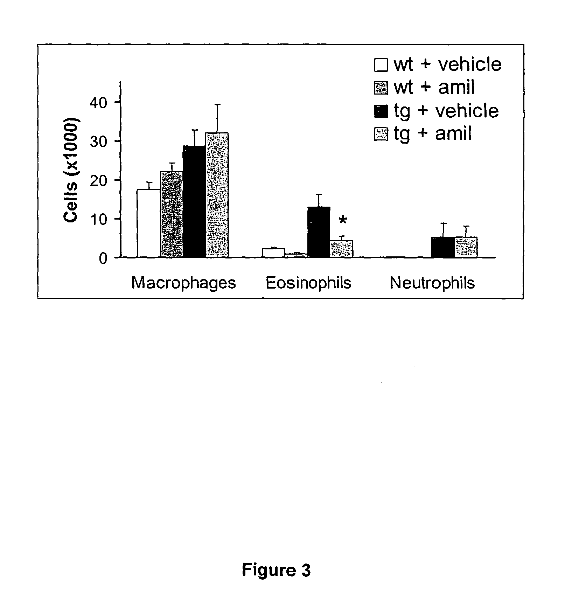

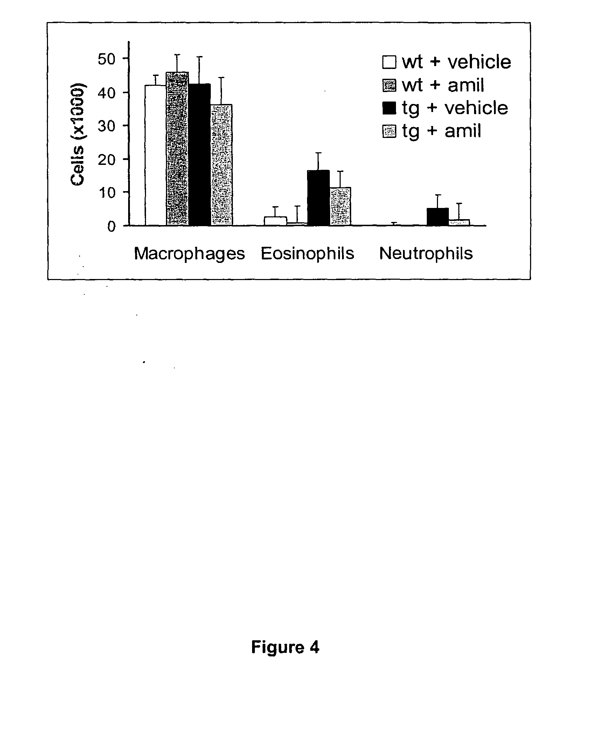

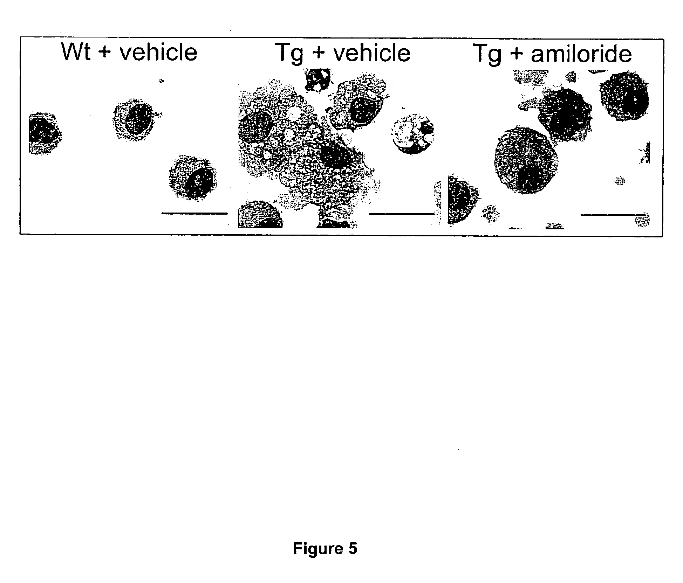

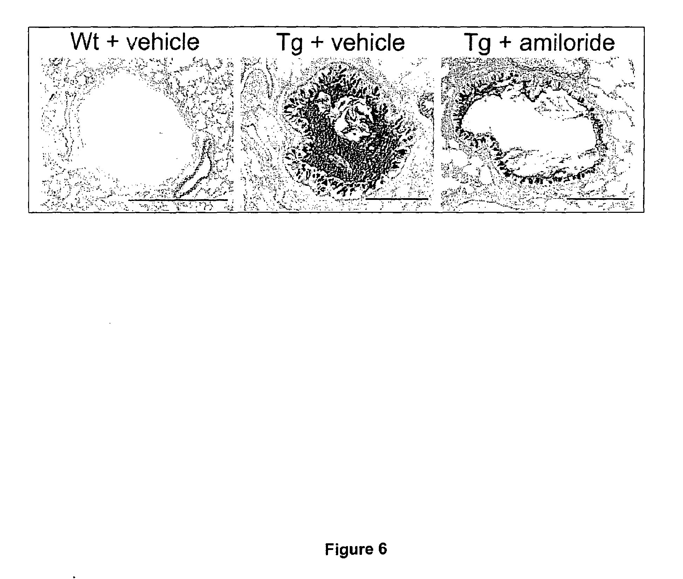

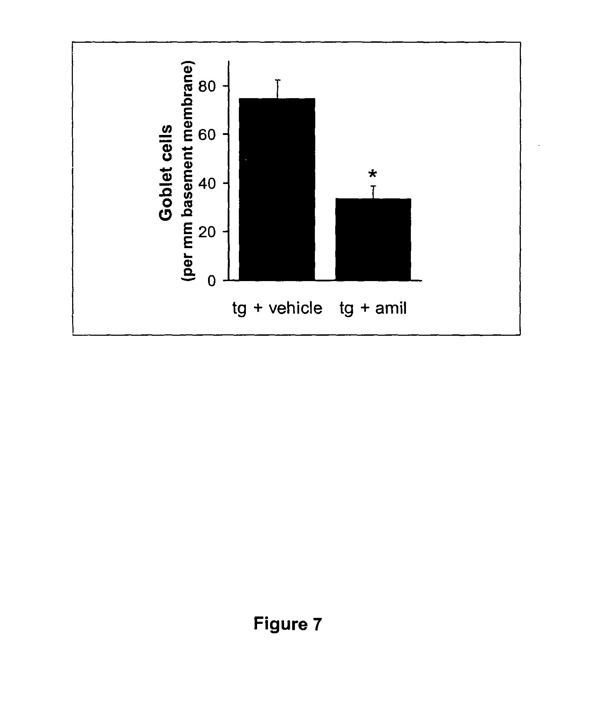

The results of said tests in β-ENaC transgenic mice showed for the first time that by an early therapy of chronic obstructive lung diseases with the sodium channel blocker amiloride significant therapeutic effect can be achieved in a living organism. Accordingly, an intrapulmonal application of amiloride in β-ENaC transgenic mice which have been treated from their first day of life on for a period of 2 weeks resulted in a significant inhibition of the pulmonal mortality (Fig. 1), a significant inhibition of the pulmonal inflammation, as determined from bronchoalveolar lavage studies (Fig. 3, 5), a significant inhibition of the mucus obstruction and goblet cell metaplasia, as determined from histopathology studies (Fig. 6, 7) as well as a significant inhibition of emphysema, as determined from histopathology and lung volume studies, when compared to vehicle treated β-ENaC transgenic mice. A later beginning of the therapy, from the fifth day of the life on, i.e. at a time point when already a progressed lung disease with airway mucus obstruction and inflammation existed in β-ENaC transgenic mice, had no therapeutic effects in the mouse model any more, i.e. mucus plugging induced mortality and pulmonary inflammation were not different in amiloride treated versus vehicle treated β-ENaC transgenic mice (Fig. 2, 4).

These results show for a first time that a chronic obstructive lung disease can be treated successfully by an early beginning of therapy with an intrapulmonary application of a specific sodium channel blocker like amiloride or a derivative thereof in a living organism. Specifically, early amiloride therapy had significant therapeutic effects on several independent clinically relevant outcomes including pulmonary mortality, airway mucus obstruction and goblet cell metaplasia, pulmonary inflammation, and development of emphysema. It is important to note that the therapeutic use can be exclusively achieved by an early therapy.

Since there has been no effective therapy for the treatment of mucus obstructions, goblet cell metaplasia, chronic pulmonary inflammation, and emphysemas of obstructive lung diseases available, the therapeutic effects which have been obtained using the above new therapeutic strategy in a mouse model represent a significant advantage when compared to therapies for the treatment of obstructive lung diseases already available. The fact that amiloride is already approved for other indications for humans will facilitate the transfer of this new therapeutic strategy to human beings.

## Claims

1. Use of at least one sodium channel blocker in the manufacture of a medicament for treating an obstructive lung disease in a patient having substantially no mucus obstruction or secondary, disease related changes of the lung.

2. The use according to claim 1, wherein the sodium channel blocker is selected from the group consisting of amiloride, amiloride analogs, P2Y2 receptor agonists, protease inhibitors, or derivatives thereof.

3. The use according to claim 2, wherein the sodium channel blocker is amiloride or a derivative thereof.

4. The use according to any one of claims 1 to 3, wherein the sodium channel blocker is used in a amount ranging from about 0.1 mg/kg body weight to about 10 mg/kg body weight.

5. The use according to any one of claims 1 to 4, wherein the obstructive lung disease is a chronic obstructive lung disease.

6. The use according to any one of claims 1 to 5, wherein the obstructive lung disease is selected from the group consisting of cystic fibrosis (CF), neonatal chronic lung disease (CLD), asthma bronchiale, and chronic bronchitis.

7. The use according to claim 6, wherein the obstructive lung disease is CF.

8. The use according to any one of claims 1 to 7, wherein the patient is a human.
